# FASCICULE DE BREVET EUROPEEN

(11) **EP 1 098 677 B1**
(45) Date de publication et mention de la délivrance du brevet: **21.09.2005**
(21) Numéro de dépôt: 99931340.6
(22) Date de dépôt: 13.07.1999
(51) Int. Cl.: A61N 1/40

(54) **PROCEDE DE TRAITEMENT COSMETIQUE DE LA PEAU ET DU CUIR CHEVELU UTILISANT DES ONDES ELECTROMAGNETIQUES ET DES HUILES ESSENTIELLES**
VERFAHREN ZUR KOSMETISCHEN BEHANDLUNG DER HAUT UND DER KOPFHAUT MIT ELEKTROMAGNETISCHEN WELLEN UND ETHERISCHEN ÖLEN
METHOD FOR COSMETIC TREATMENT OF THE SKIN AND THE SCALP USING ELECTROMAGNETIC WAVES AND ESSENTIAL OILS

(30) Priorité: 17.07.1998 FR 9809522
(43) Date de publication de la demande: 16.05.2001
(73) Titulaire: BX3 General Labs S.A., 1815 Clarens Montreux (CH)
(72) Inventeur: MAZAURY, André, 13250 Saint-Chamas (FR); BELLISI, Vincente, 13250 Saint-Chamas (FR)
(74) Mandataire: Santarelli
(86) Numéro de dépôt international: PCT/FR1999/001712
(87) Numéro de publication internationale: WO 2000/003762

(56) Documents cités:
- EP-A- 0 497 672
- EP-A- 0 655 261
- WO-A-94/21326
- FR-A- 2 454 817
- FR-A- 2 665 366
- FR-A- 2 706 131

## Description

La présente invention concerne un nouveau procédé de traitement cosmétique de la peau, du cuir chevelu et de leurs dérivés utilisant des ondes électromagnétiques à haute fréquence pulsées à effet athermique et des huiles essentielles.

Traditionnellement, les problèmes de la peau, du cuir chevelu et des cheveux sont traités par toutes sortes de principes actifs parmi lesquels on peut citer l'allantoïne, les alpha-hydroxy acides, le coaltar, le minoxidil, se présentant sous toute une variété de formes galéniques telles que les crèmes, les laits, les lotions, les émulsions eau dans huile ou huile dans eau, les shampooings, etc.....

Certains problèmes sont également traités comme décrit dans FR-A-2 665 366. Selon ce document, on utilise un appareil générateur de courant basse fréquence au contact avec la peau à l'emplacement à traiter, produisant un courant alternatif de quelques millionièmes d'ampère d'intensité pour pratiquer un micro-massage de la peau. Le boîtier de l'appareil forme une électrode et l'autre électrode est appliquée sur la peau de l'utilisateur, ce qui ferme le circuit pour réaliser le massage mécanique. Selon ce document, le massage électrique produit par lui-même un effet cosmétique puisque les rides s'atténueraient et les pores de la peau se resserreraient ; en outre, les produits, crèmes ou huiles essentielles, pourraient pénétrer plus profondément dans la peau et ainsi décupler leur action, comme pourrait le faire un massage manuel. Ces suppositions qu'un massage électrique peut remplacer un massage manuel conventionnel ne sont cependant étayées par aucune preuve.

Ces problèmes sont également traités par un certain nombre d'appareillages parmi lesquels on peut citer les lasers, les appareils délivrant des courants faradiques ou galvaniques, des appareils délivrant de la vapeur d'eau, ou des émetteurs d'ondes électromagnétiques à haute fréquence pulsées athermiques comme décrit dans EP-0 497 672.

On connaît également par FR-A-2 665 366 un appareil d'électrostimulation autonome produisant un courant faradique alternatif basse fréquence travaillant en circuit fermé en utilisant deux électrodes directement en contact avec la peau, qui produirait ainsi un micro massage de la peau.

En particulier, on recherche toujours un procédé efficace pour lutter contre la chute des cheveux. En effet, tous les traitements actuels, même les plus récents comme ceux à base de minoxidil, se sont avérés décevants.

Or, après de longues études, la demanderesse a découvert qu'un traitement par ondes électromagnétiques à haute fréquence pulsées à effet athermique utilisé selon certains paramètres amplifiait considérablement et d'une manière inattendue les effets propres d'un certain nombre d'huiles essentielles.

C'est pourquoi la présente invention a pour objet un procédé d'amplification avec effet synergique des effets conventionnels d'une huile essentielle à propriétés bénéfiques pour la peau, le cuir chevelu, les ongles et les cheveux et d'ondes électromagnétiques à hautes fréquences pulsées, caractérisé en ce que l'on applique sur la zone à traiter l'huile essentielle ou le mélange d'huiles essentielles désiré, et soumet ladite zone à l'action d'ondes électromagnétiques à hautes fréquences pulsées d'une fréquence comprise entre 1 MHz et 300 MHz, avec un espacement de 0,1 à 400 millisecondes entre chaque pulsation d'ondes, et avec une puissance de 10⁻⁶ W à 2 W.

Les huiles essentielles utilisables peuvent être par exemple, en ce qui concerne les problèmes de chute de cheveux, une ou plusieurs huiles essentielles choisies parmi les huiles essentielles, de bay St Thomas, de bouleau, de cade, de camomille, de cannelle, de cèdre, de citron, de cyprès, de géranium, de laurier, de lavande, d'orange, de patchouli, de pin, de romarin, de santal, de sauge, de serpolet, de thuya, de thym, ou d'ylang-ylang. On utilise de préférence des huiles essentielles de bay St Thomas, de bouleau, de cade, de cannelle, de cèdre, de cyprès, de laurier, de patchouli, de romarin, de santal, de sauge, de thuya, de thym, ou d'ylang-ylang, notamment des huiles essentielles de bay St Thomas, de bouleau, de cade, de cannelle, de cèdre, de laurier, de patchouli, de romarin, de thuya, de thym, ou d'ylang-ylang, tout particulièrement des huiles essentielles de bouleau, de cannelle, de cèdre, de laurier, de patchouli, de romarin, de thuya, de thym, ou d'ylang-ylang.

Pour les problèmes de dermatoses cutanées du ressort de la cosmétique, on peut utiliser par exemple une ou plusieurs huiles essentielles choisies parmi les huiles essentielles d'aspic, de basilic, de benjoin, de bergamote, de bois de rose, de bouleau, de cade, de cajeput, de camomille, de cannelle, de carotte, de cèdre, de citron, de copahu, de cyprès, d'élémi, d'eucalyptus, de gaultherie, de genièvre, de géranium, de girofle, de gurjum, d'hélichryse, d'hysope, de laurier, de lavande, de lavandin, de marjolaine, de mélisse, de myrrhe, de myrte, de niaouli, d'orange bigarade, d'orange, d'origan, de palmarosa, de patchouli, de pin, de romarin, de rose, de santal, de sarriette, de sassafras, de sauge, de serpolet, de styrax, de thuya, de thym, de vétiver, d'ylang-ylang et des baumes du Canada, du Pérou et de Tolu. On utilise de préférence des huiles essentielles de benjoin, de bois de rose, de bouleau, de cade, de cajeput, de camomille, de carotte, de cèdre, de citron, de copahu, de géranium, de girofle, de gurjum, de laurier, de lavande, de marjolaine, de myrte, de niaouli, d'origan, de palmarosa, de patchouli, de santal, de sauge et le baume du Pérou, notamment des huiles essentielles de benjoin, de bois de rose, de cade, de cajeput, de carotte, de cèdre, de citron, de copahu, de géranium, de girofle, de gurjum, de lavande, de marjolaine, de niaouli, de palmarosa, de patchouli et de sauge, tout particulièrement des huiles essentielles de benjoin, de bois de rose, de cade, de carotte, de géranium, de lavande, de marjolaine, de niaouli, de patchouli et de sauge.

Pour les problèmes de rides, on peut utiliser par exemple une ou plusieurs huiles essentielles choisies parmi les huiles essentielles d'amande amère, d'aspic, de basilic, de benjoin, de bois de rose, de cade, de cajeput, de camomille, de cannelle, de carotte, de cèdre, de citron, de copahu, de cyprès, d'élémi, d'eucalyptus, de genièvre, de géranium, de girofle, d'hélichryse, d'hysope, de lavande, de limette, de macis, de muscade, de niaouli, d'orange bigarade, d'orange, d'origan, de palmarosa, de pamplemousse, de patchouli, de romarin, de rose, de santal, de sarriette, de sauge, de thym, d'ylang-ylang, et des baumes du Canada, du Pérou ou de Tolu. On utilise de préférence des huiles essentielles de basilic, de benjoin, de bois de rose, de cajeput, de camomille, de carotte, de cèdre, de cyprès, de géranium, de lavande, de niaouli, d'orange bigarade, d'origan, de palmarosa, de patchouli, de romarin, de santal, de sauge, de thym et d'ylang-ylang, notamment de basilic, de bois de rose, de cajeput, de camomille, de carotte, de cyprès, de géranium, de lavande, de niaouli, d'orange bigarade, de palmarosa, de patchouli, de romarin, de sauge, de thym et d'ylang-ylang et tout particulièrement de basilic, de bois de rose, de carotte, de cyprès, de géranium, de lavande, d'orange bigarade, de palmarosa, de patchouli, de romarin, de sauge et de thym.

Pour les problèmes de peaux rosées, on peut utiliser par exemple une ou plusieurs huiles essentielles choisies parmi les huiles essentielles d'amande amère, d'aspic, de basilic, de benjoin, de bois de rose, de cade, de cajeput, de camomille, de cannelle, de carotte, de cèdre, de citron, de copahu, de cyprès, d'élémi, d'eucalyptus, de genièvre, de géranium, de girofle, d'hélichryse, d'hysope, de lavande, de limette, de macis, de muscade, de niaouli, d'orange bigarade, d'orange, d'origan, de palmarosa, de pamplemousse, de patchouli, de romarin, de rose, de santal, de sarriette, de sauge, de thym et d'ylang-ylang et des baumes du Canada, du Pérou et de Tolu. On utilise de préférence des huiles essentielles de benjoin, de bois de rose, de camomille, de carotte, de cèdre, de citron, de cyprès, de géranium, d'hélichryse, de lavande, de niaouli, d'orange bigarade, d'origan, de palmarosa, de patchouli, de romarin, de rose, de santal, de sauge, de thym et d'ylang-ylang et du baume du Pérou, notamment de benjoin, de bois de rose, de camomille, de carotte, de cèdre, de citron, de cyprès, de géranium, d'hélichryse, de lavande, d'orange bigarade, de palmarosa, de patchouli, de romarin, de sauge et de thym, particulièrement de bois de rose, de carotte, de cèdre, de citron, de cyprès, d'hélichryse, de lavande, d'orange bigarade, de palmarosa, de patchouli, de romarin, de sauge et de thym et tout particulièrement de bois de rose, de cèdre, de citron, de cyprès, d'hélichryse, de lavande, d'orange bigarade, de palmarosa, de patchouli, de romarin, de sauge et de thym.

Pour les problèmes de seins comme pour le raffermissement de la poitrine, on peut utiliser par exemple une ou plusieurs huiles essentielles choisies parmi les huiles essentielles d'amande amère, d'aspic, de basilic, de benjoin, de bois de rose, de cade, de cajeput, de camomille, de cannelle, de carotte, de cèdre, de citron, de copahu, de cyprès, d'élémi, d'eucalyptus, de genièvre, de géranium, de girofle, d'hélichryse, d'hysope, de lavande, de limette, de macis, de muscade, de niaouli, d'orange bigarade, d'orange, d'origan, de palmarosa, de pamplemousse, de patchouli, de romarin, de rose, de santal, de sarriette, de sauge, de thym, et d'ylang-ylang et des baumes du Canada, du Pérou et de Tolu. On utilise de préférence des huiles essentielles d'amande amère, de benjoin, de bois de rose, de camomille, de carotte, de cèdre, de citron, de cyprès, d'eucalyptus, de girofle, d'hysope, de lavande, de limette, de macis, de muscade, d'orange bigarade, d'origan, de palmarosa, de patchouli, de romarin, de santal, de sauge, notamment d'amande amère, de bois de rose, de carotte, de cèdre, de citron, de cyprès, d'eucalyptus, de girofle, d'hysope, de limette, de macis, de muscade, d'orange bigarade, de palmarosa, de patchouli, de romarin, de santal, de sauge et tout particulièrement d'amande amère, de bois de rose, de cèdre, de citron, d'eucalyptus, de girofle, d'hysope, de limette, de macis, de muscade, d'orange bigarade, de palmarosa, de romarin et de santal.

Ces huiles essentielles, de préférence naturelles, peuvent être utilisées pures ou diluées par exemple dans des huiles végétales, notamment de rose musquée, de sésame, de carthame, de soja, de tournesol, de pépin de raisin, de germe de blé, d'olive, d'onagre ou de bourrache. La dilution peut aller au 1/2, voire au 1/4, voire au 1/10, voire même au 1/20ème. Des faibles concentrations comme 5 ou 10 % voire 30 % sont utilisées de préférence pour des zones où la peau est sensible et les fortes concentrations pour des zones telles que le cuir chevelu non irrité.

On combine avantageusement de 4 à 20 huiles essentielles et de préférence de 6 à 15 huiles essentielles.

Ces huiles essentielles se trouvent couramment dans le commerce et peuvent être obtenues par exemple auprès de la Société ADRIAN à Marseille (France), ou auprès de la COOPER à Melun (France).

Les huiles végétales peuvent être obtenues par exemple auprès de la Société SICTIA à Marseille.

Les ondes électromagnétiques haute fréquence pulsées à effet athermique ci-dessus peuvent être obtenues par exemple à l'aide du dispositif décrit dans EP-A-0 497 672.

La puissance d'émission du générateur d'ondes peut aller par exemple de 10⁻⁶ W à 2 W. En cas d'application directe et localisée à la zone à traiter, on peut se contenter d'une puissance de 0,2 à 1 mW par exemple. La puissance d'émission est de préférence uniformément répartie sur la surface de la zone à traiter.

Dans des conditions préférentielles de réalisation, l'émission des ondes est réalisée à l'aide d'une ou plusieurs antennes installées sur un dispositif servant de support à la ou aux antennes, lesdites antennes étant espacées de la zone à traiter, de moins de 50 cm, de préférence de 0,5 à 15 cm, notamment de 1 à 5 cm, tout particulièrement de 1,5 à 3 cm. C'est ainsi que par exemple dans le cas d'un émetteur portatif, la ou les antennes seront espacées de la zone à traiter par l'intermédiaire par exemple d'un système d'entretoises espaçant l'antenne de la zone à traiter, et prenant appui par exemple à l'extérieur de la zone à traiter. C'est pourquoi par exemple dans le traitement de la calvitie, on peut notamment utiliser un casque prenant appui au niveau du front et sur la nuque (ces appuis constituant le système d'entretoises), le casque servant de support à une ou plusieurs antennes espacées du crâne.

La forme de l'antenne peut être par exemple plate comme montré ci-après dans la partie expérimentale, mais dans d'autres conditions préférentielles, la forme de l'antenne épouse celle de la zone à traiter afin de transmettre une émission régulière d'ondes électromagnétiques à toute ladite zone. Il est à noter que l'absence de contact avec la zone à traiter permet d'éviter les risques d'électrocution.

La surface de l'antenne ou des antennes peut être par exemple de 10 à 600 cm², mais dans des conditions préférentielles, la surface va de 50 à 300 cm².

Le nombre d'antennes du même émetteur peut être par exemple de 1 à 9 mais dans des conditions préférentielles, ce nombre va de 1 à 4. On peut bien sûr utiliser de nombreuses antennes individuelles par exemple de 10 à 20 de petite surface notamment pour ne localiser l'émission qu'à l'emplacement désiré, les autres antennes restant inactives.

La fréquence d'émission peut être par exemple de 1 à 300 MHz, mais dans des conditions préférentielles, la fréquence d'émission va de 6 à 50 MHz.

L'espacement entre deux pulsions peut être par exemple de 0,1 à 200 ms, mais dans des conditions préférentielles, cet espacement va de 0,4 à 200 ms.

La durée d'une pulsion peut être par exemple de 0,1 à 200 ms, mais dans des conditions préférentielles, cette durée va de 0,4 à 200 ms.

Dans des conditions préférentielles de mise en oeuvre du procédé ci-dessus décrit, la durée d'une pulsion et l'espacement entre deux pulsions sont identiques, ce qui simplifie la construction.

La puissance d'émission peut être par exemple de 10⁻⁶ W à 2 W, mais dans des conditions préférentielles, l'intensité d'émission va de 0,2 à 1 mW.

L'émission des ondes sur la zone à traiter est effectuée de préférence moins de 72 heures après l'application des huiles essentielles, notamment moins de 3 heures, particulièrement moins de 1 heure, tout particulièrement juste après l'application des huiles essentielles.

Selon les études de la demanderesse, les propriétés notamment antiseptiques, bactériostatiques, bactéricides, antivirales, anti-mycosiques, cicatrisantes, stimulantes et rééquilibrantes fonctionnelles, catalytiques, bio-psychiques, et physiques des huiles essentielles proviendraient de leur possibilité de donner ou de recevoir de l'énergie électronique grâce à leur structure moléculaire. En effet, les ondes constituent un moyen de locomotion de l'énergie vibratoire de ses deux composantes, électrique et magnétique. Or, les ondes électromagnétiques à haute fréquence pulsées à effet athermique prépareraient l'action des huiles essentielles en agissant sur la réceptivité des cellules, et en plus en favorisant la réceptivité et la faculté d'émission des molécules notamment aromatiques, présentes dans les huiles essentielles, en provoquant ainsi une synergie d'effets inattendue.

Le procédé selon l'invention permet donc d'amplifier considérablement les effets des huiles essentielles utilisées isolément.

Il trouve donc son application notamment dans les divers traitements cosmétiques à base d'huiles essentielles, bien connus de l'état de la technique.

La présente demande a encore pour objet un kit de traitement cosmétique de la peau, du cuir chevelu et de leurs dérivés, pour la mise en oeuvre du procédé ci-dessus, caractérisé en ce qu'il comprend un ou plusieurs mélanges d'huiles essentielles, et un générateur d'ondes électromagnétiques à haute fréquence pulsées athermiques, notamment capable d'émettre des ondes ayant les caractéristiques ci-dessus, éventuellement accompagné d'instructions pour l'utilisation conjointe d'huiles essentielles en vue d'en amplifier les effets. Ce générateur peut être de type portatif comme montré ci-après dans la partie expérimentale, ou de type « professionnel », c'est-à-dire installé sur une potence comme décrit dans EP-A-0 497 672.

La présente demande a enfin pour objet
- une méthode de traitement des problèmes de chute de cheveux,
- une méthode de traitement des problèmes de dermatoses cutanées du ressort de la cosmétique,
- une méthode de traitement des problèmes de rides,
- une méthode de traitement des problèmes de peaux rosées,
- une méthode de traitement des problèmes de seins comme pour le rafermissement de la poitrine,
dans lesquelles on utilise des ondes électromagnétiques à haute fréquence pulsées athermiques pour amplifier les effets (avec effet synergique), d'huiles essentielles.

Les exemples qui suivent illustrent la présente invention.
La figure 1 représente schématiquement une vue latérale en élévation et coupe partielle d'un casque portatif émetteur d'ondes électromagnétiques à haute fréquence pulsées installé sur le crâne d'un individu.
La figure 2 est une vue schématique de dessus d'un casque émetteur de la figure 1, éclatée au niveau de l'antenne.

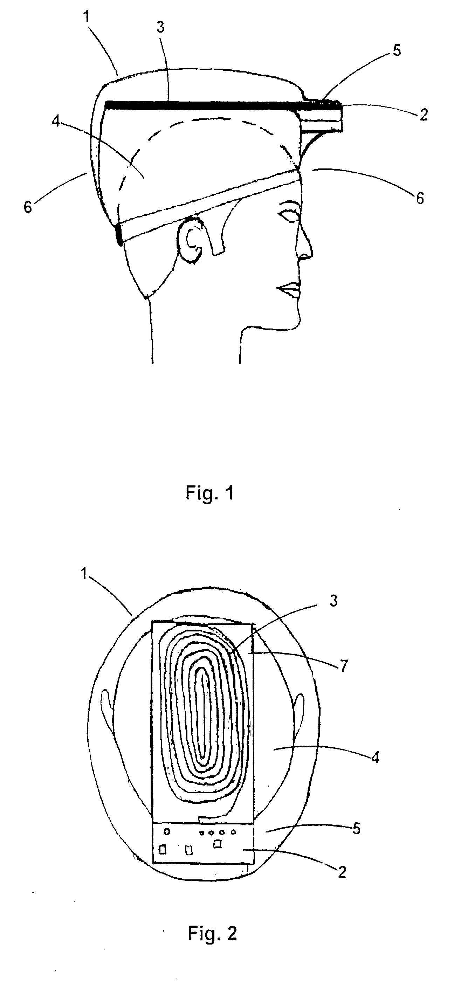

Sur la figure 1, on peut observer le casque 1 servant de support à une antenne émettrice 3 de forme plane et constituée par la plaque d'un circuit imprimé en spirale placé à une distance de 1 cm à quelques centimètres du crâne. Ce casque 1 inclut le boîtier de commande 5 dans lequel se trouvent la partie électronique 2 et une batterie et en surface les boutons de sélection de fréquence, de puissance, de temporisation et de mise en route. L'antenne 3 est espacée du crâne 4 à l'aide des côtés inférieurs 6 du casque servant d'entretoise.

Sur la figure 2, on peut observer plus particulièrement le dessin en spirale de l'antenne 3 sur son circuit imprimé 7 solidaire du casque et les boutons de sélection. Le circuit imprimé a une largeur d'environ 10 cm. L'antenne est spiralée et comprend environ 20 spires.

L'absence de contact de l'antenne avec la zone à traiter permet d'éviter les risques d'électrocution.

### Exemple 1 : Mélange d'huiles essentielles pour le traitement de la chute des cheveux.

| Huiles essentielles | Pourcentage |
|---|---|
| Bouleau | 6,67 |
| Cannelle | 13,33 |
| Cèdre | 13,33 |
| Laurier | 13,33 |
| Patchouli | 5,00 |
| Romarin | 6,67 |
| Thuya | 25,00 |
| Thym | 10,00 |
| Ylang-ylang | 6,67 |
| TOTAL | 100 % |

### Exemple 2 : Mélange d'huiles essentielles diluées pour le traitement cosmétique des dermatoses

| Huiles essentielles | Pourcentage | Huiles végétales | Pourcentage |
|---|---|---|---|
| Benjoin | 5,00 | Olive | 16,67 |
| Bois de rose | 3,33 | Sésame | 16,67 |
| Cade | 3,33 | Carthame | 16,67 |
| Carotte | 5,00 | | |
| Géranium | 5,00 | | |
| Lavande | 6,67 | | |
| Marjolaine | 3,33 | | |
| Niaouli | 5,00 | | |
| Patchouli | 6,67 | | |
| Sauge | 6,67 | | |
| TOTAL | 50 % | TOTAL | 50 % |

### Exemple 3 : Mélange d'huiles essentielles diluées pour le traitement cosmétique des peaux rosées

| Huiles essentielles | Pourcentage | Huiles végétales | Pourcentage |
|---|---|---|---|
| Bois de rose | 0,67 | Bourrache | 11,00 |
| Cèdre | 2,00 | Germe de blé | 12,00 |
| Citron | 1,33 | Carthame | 11,00 |
| Cyprès | 2,67 | Soja | 11,00 |
| Hélichryse | 2,67 | Tournesol | 11,00 |
| Lavande | 1,33 | Pépin de raisin | 24,00 |
| Orange bigarade | 1,33 | | |
| Palmarosa | 2,67 | | |
| Patchouli | 2,00 | | |
| Romarin | 2,00 | | |
| Sauge | 0,67 | | |
| Thym | 0,67 | | |
| TOTAL | 20 % | TOTAL | 80 % |

### Exemple 4 : Mélange d'huiles essentielles diluées pour le traitement cosmétique des rides

| Huiles essentielles | Pourcentage | Huiles végétales | Pourcentage |
|---|---|---|---|
| Basilic | 1,33 | Onagre | 11,00 |
| Bois de rose | 1,33 | Rose musquée | 11,00 |
| Carotte | 2,00 | Carthame | 11,00 |
| Cyprès | 1,33 | Sésame | 11,00 |
| Géranium | 1,33 | Germe de blé | 12,00 |
| Lavande | 1,33 | Pépin de raisin | 24,00 |
| Orange bigarade | 2,00 | | |
| Palmarosa | 2,70 | | |
| Patchouli | 2,00 | | |
| Romarin | 2,00 | | |
| Sauge | 1,33 | | |
| Thym | 1,33 | | |
| TOTAL | 20 % | TOTAL | 80 % |

### Exemple 5 : Mélange d'huiles essentielles diluées pour le raffermissement des seins

| Huiles essentielles | Pourcentage | Huiles végétales | Pourcentage |
|---|---|---|---|
| Amande amère | 2,50 | Pépin de raisin | 22,40 |
| Bois de rose | 0,84 | Germe de blé | 13,15 |
| Cèdre | 1,66 | Carthame | 13,15 |
| Citron | 1,66 | Soja | 13,15 |
| Eucalyptus | 2,50 | Tournesol | 13,15 |
| Girofle | 1,66 | | |
| Hysope | 2,50 | | |
| Limette | 0,84 | | |
| Macis | 0,84 | | |
| Muscade | 1,66 | | |
| Orange bigarade | 0,84 | | |
| Palmarosa | 3,34 | | |
| Romarin | 1,66 | | |
| Santal | 2,50 | | |
| TOTAL | 25 % | TOTAL | 75 % |

### Exemple 6 : Mise en évidence de l'effet combiné du mélange d'huiles essentielles de l'exemple 1 et d'ondes électromagnétiques à haute fréquence pulsées à effet athermique

On a constitué un groupe de 30 personnes présentant une chute de cheveux de type androgénétique, et dont la surface de la calotte allait de 0 à 80 cm². On a soumis un premier tiers de ces personnes à un massage conventionnel manuel de la zone à traiter avec 0,5 ml du mélange d'huiles essentielles de l'exemple 1 trois fois par semaine pendant trois mois.

Un second tiers a été soumis à un traitement pendant une demi-heure, à l'aide du casque décrit aux figures 1 et 2, deux fois par semaine pendant trois mois. L'émission du casque correspondait aux caractéristiques suivantes :
- Surface de l'antenne : 200 cm²
- Fréquence d'émission : 10 MHz
- Espacement entre deux pulsions : 0,625 ms
- Durée d'une pulsion : 0,625 ms
- Puissance : 1 mW
- Espacement moyen entre les antennes et la zone à traiter : 2 cm.

Le dernier tiers a été soumis au traitement ci-dessus à base d'huiles essentielles, suivi, 1 minute après, par le traitement ci-dessus à base d'ondes électromagnétiques à haute fréquence pulsées athermiques.

Les résultats obtenus sont les suivants :
- 1er tiers :: arrêt de la chute anormale et amélioration de la qualité du cheveu dans 70 % des cas au bout d'un mois avec apparition d'un duvet dans 10 % des cas au bout de trois mois.
- 2ème tiers :: amélioration de la qualité des cheveux, stabilisation de la chute anormale dans 50 % des cas au bout de deux mois.
- 3ème tiers :: arrêt de la chute anormale et amélioration de la qualité du cheveu dans 80 % des cas au bout d'un mois avec repousse dans 50 % des cas au bout de deux mois.

La synergie des effets se traduit par des résultats positifs plus nombreux et plus rapides sur
- l'arrêt de la chute et l'amélioration des cheveux :
   - 80 % au lieu de 70 % avec les huiles essentielles seules et 50 % avec les ondes seules ;
   - 1 mois au lieu de 2 avec les ondes seules
- la repousse :
   - 50 % au lieu de 10 % avec les huiles essentielles seules et 0 % avec les ondes seules ;
   - 2 mois au lieu de 3 avec les huiles essentielles seules

De plus le cheveu qui repousse ne reste pas au stade de duvet.

### Exemple 7 : Mise en évidence de l'effet combiné du mélange d'huiles essentielles de l'exemple 1 et d'ondes électromagnétiques à haute fréquence pulsées athermiques

On a pris un groupe de 50 personnes présentant une chute de cheveux de type androgénétique, et dont la surface de la calotte allait de 0 à 80 cm² que l'on a soumis au traitement combiné ci-dessus.

Une amélioration a pu être observée sur 41 personnes au bout d'un mois de traitement. Cette amélioration de la qualité se distinguait par l'épaississement des cheveux, la diminution voire la disparition de l'état de pelliculaire et/ou de l'hyperséborrhée à l'origine de la chute des cheveux et un arrêt de la chute excessive. Après deux mois de traitement, une repousse des cheveux a été observée dans environ 50 % des cas.

On a donc obtenu la confirmation des résultats déjà obtenus mettant en évidence l'indéniable complémentarité de la combinaison huiles essentielles - ondes électromagnétiques à hautes fréquences pulsées sur l'arrêt de la chute excessive, l'amélioration des cheveux et la repoussse.

### Exemple 8: Traitement de dermatoses du cuir chevelu de type eczémateux

On a traité un certain nombre de problèmes de dermatose du cuir chevelu de type eczémateux en procédant comme indiqué à l'exemple 4, mais en utilisant le mélange d'huiles essentielles de l'exemple 2, et en traitant une demi-heure trois fois par semaine à l'aide des ondes électromagnétiques et application du mélange d'huiles essentielles tous les jours, et ce pendant trois mois.

Dès les premières séances, une nette amélioration a été observée, avec disparition totale sur 37 des personnes au bout d'une vingtaine de jours.

Ce type de problème étant souvent lié à des phénomènes psychosomatiques et pouvant revenir en cas de stress, ces soins doivent être poursuivis ou repris dès la réapparition des symptômes, afin de les faire disparaître très rapidement.

## Revendications

1. Un procédé non thérapeutique d'amplification avec effet synergique des effets conventionnels d'une huile essentielle à propriétés bénéfiques pour la peau, le cuir chevelu, les ongles et les cheveux et d'ondes électromagnétiques à hautes fréquences pulsées, **caractérisé en ce que** l'on applique sur la zone à traiter l'huile essentielle ou le mélange d'huiles essentielles désiré, et soumet ladite zone à l'action d'ondes électromagnétiques à hautes fréquences pulsées athermiques, d'une fréquence comprise entre 1 MHz et 300 MHz, avec un espacement de 0,1 à 400 millisecondes entre chaque pulsation d'ondes, et avec une puissance de 10⁻⁶ W à 2 W.

2. Un procédé selon la revendication 1, **caractérisé en ce que** la puissance d'émission du générateur d'ondes utilisée va de 0,2 à 1 mW.

3. Un procédé selon l'une des revendications 1 et 2, **caractérisé en ce que** l'émission des ondes est réalisée à l'aide d'une ou plusieurs antennes installées sur un dispositif servant de support à la ou aux antennes, lesdites antennes étant espacées de la zone à traiter de 0,5 à 15 cm.

4. Un procédé selon l'une des revendications 1 à 3, **caractérisé en ce que** la surface de l'antenne ou des antennes va de 50 à 300 cm².

5. Un procédé selon l'une des revendications 1 à 4, **caractérisé en ce que** l'émission des ondes est effectuée moins de 3 heures après l'application des huiles essentielles.

6. Un procédé selon l'une des revendications 1 à 5, **caractérisé en ce que** l'on traite les problèmes de chute de cheveux, en utilisant une ou plusieurs huiles essentielles choisies parmi les huiles essentielles de bay St Thomas, de bouleau, de cade, de camomille, de cannelle, de cèdre, de citron, de cyprès, de géranium, de laurier, de lavande, d'orange, de patchouli, de pin, de romarin, de santal, de sauge, de serpolet, de thuya, de thym, ou d'ylang-ylang.

7. Un procédé selon l'une des revendications 1 à 5, **caractérisé en ce que** l'on traite les problèmes de rides, en utilisant une ou plusieurs huiles essentielles choisies parmi les huiles essentielles d'amande amère, d'aspic, de basilic, de benjoin, de bois de rose, de cade, de cajeput, de camomille, de cannelle, de carotte, de cèdre, de citron, de copahu, de cyprès, d'élémi, d'eucalyptus, de genièvre, de géranium, de girofle, d'hélichryse, d'hysope, de lavande, de limette, de macis, de muscade, de niaouli, d'orange bigarade, d'orange, d'origan, de palmarosa, de pamplemousse, de patchouli, de romarin, de rose, de santal, de sarriette, de sauge, de thym, d'ylang-ylang, et des baumes du Canada, du Pérou ou de Tolu.

8. Un procédé selon l'une des revendications 1 à 5, **caractérisé en ce que** l'on traite les problèmes de peaux rosées, en utilisant une ou plusieurs huiles essentielles choisies parmi les huiles essentielles d'amande amère, d'aspic, de basilic, de benjoin, de bois de rose, de cade, de cajeput, de camomille, de cannelle, de carotte, de cèdre, de citron, de copahu, de cyprès, d'élémi, d'eucalyptus, de genièvre, de géranium, de girofle, d'hélichryse, d'hysope, de lavande, de limette, de macis, de muscade, de niaouli, d'orange bigarade, d'orange, d'origan, de palmarosa, de pamplemousse, de patchouli, de romarin, de rose, de santal, de sarriette, de sauge, de thym et d'ylang-ylang et des baumes du Canada, du Pérou et de Tolu.

9. Un procédé selon l'une des revendications 1 à 5, **caractérisé en ce que** l'on traite les problèmes des seins comme pour le raffermissement de la poitrine, en utilisant une ou plusieurs huiles essentielles choisies parmi les huiles essentielles d'amande amère, d'aspic, de basilic, de benjoin, de bois de rose, de cade, de cajeput, de camomille, de cannelle, de carotte, de cèdre, de citron, de copahu, de cyprès, d'élémi, d'eucalyptus, de genièvre, de géranium, de girofle, d'hélichryse, d'hysope, de lavande, de limette, de macis, de muscade, de niaouli, d'orange bigarade, d'orange, d'origan, de palmarosa, de pamplemousse, de patchouli, de romarin, de rose, de santal, de sarriette, de sauge, de thym, et d'ylang-ylang et des baumes du Canada, du Pérou et de Tolu.

10. Un kit de traitement cosmétique de la peau, du cuir chevelu et de leurs dérivés pour la mise en oeuvre du procédé selon l'une des revendications 1 à 9, **caractérisé en ce qu'**il comprend un ou plusieurs mélanges d'huiles essentielles, et un générateur (1) d'ondes électromagnétiques à haute fréquence pulsées athermiques capable d'émettre une fréquence comprise entre 1 MHz et 300 MHz, avec un espacement de 0,1 à 400 millisecondes entre chaque pulsation d'ondes, avec une puissance de 10⁻⁶ W à 2 W.

11. Un kit de traitement cosmétique de la peau, du cuir chevelu et de leurs dérivés selon la revendication 10, **caractérisé en ce qu'**il comprend un ou plusieurs mélanges d'huiles essentielles, un générateur (1) d'ondes électromagnétiques à haute fréquence pulsées athermiques capable d'émettre une fréquence comprise entre 1 MHz et 300 MHz, avec un espacement de 0,1 à 400 millisecondes entre chaque pulsation d'ondes, avec une puissance de 10⁻⁶ W à 2 W, et des instructions pour l'utilisation conjointe d'huiles essentielles en vue d'en amplifier les effets.

## Patentansprüche

1. Nicht-therapeutisches Verstärkungsverfahren mit synergistischer Wirkung der konventionellen Wirkungen eines essentiellen Öls mit günstigen Eigenschaften für die Haut, die behaarte Kopfhaut, die Nägel und die Haare und von gepulsten hochfrequenten elektromagnetischen Wellen, **dadurch gekennzeichnet, dass** man auf die zu behandelnde Zone das essentielle Öl oder das Gemisch essentieller Öle aufträgt und man die Zone der Wirkung athermischer gepulster hochfrequenter elektromagnetischer Wellen einer Frequenz zwischen 1 MHz und 300 MHz mit einem Abstand von 0,1 bis 400 ms zwischen jeder Wellenpulsation und mit einer Leistung von 10⁻⁶ W bis 2 W unterwirft.

2. Verfahren nach Anspruch 1, **dadurch gekennzeichnet, dass** die Emissionsleistung des verwendeten Wellengenerators von 0,2 bis 1 mW reicht.

3. Verfahren nach einem der Ansprüche 1 und 2, **dadurch gekennzeichnet, dass** die Emission der Wellen mit Hilfe einer oder mehrerer Antennen verwirklicht wird, welche auf einer Vorrichtung installiert sind, die als Träger für die Antenne(n) dient, wobei die Antennen 0,5 bis 15 cm von der zu behandelnden Zone beabstandet sind.

4. Verfahren nach einem der Ansprüche 1 bis 3, **dadurch gekennzeichnet, dass** die Oberfläche der Antenne oder der Antennen von 50 bis 300 cm² reicht.

5. Verfahren nach einem der Ansprüche 1 bis 4, **dadurch gekennzeichnet, dass** die Emission der Wellen weniger als 3 Stunden nach Anwendung bzw. Auftragung der essentiellen Öle durchgeführt wird.

6. Verfahren nach einem der Ansprüche 1 bis 5, **dadurch gekennzeichnet, dass** man die Haarausfall-Probleme behandelt, indem man ein essentielles Öl oder mehrere essentielle Öle verwendet, das/die unter den essentiellen Ölen Bay St. Thomas-Öl, Birkenholzöl, Cadeöl, Kamillenöl, Zimtöl, Zedernöl, Zitronenöl, Zypressenöl, Geranienöl, Lorbeeröl, Lavendelöl, Orangenöl, Patschuliöl, Fichtenöl, Rosmarinöl, Sandelholzöl, Salbeiöl, Feldthymianöl, Thujenöl, Thymianöl, Ylang-Ylang-Öl ausgewählt ist/sind.

7. Verfahren nach einem der Ansprüche 1 bis 5, **dadurch gekennzeichnet, dass** man Faltenprobleme behandelt, indem man ein essentielles Öl oder mehrere essentielle Öle verwendet, das/die unter den essentiellen Ölen Bittermandelöl, Lavendelöl, Basilikumöl, Benzoeöl, Rosenholzöl, Cadeöl, Eukalyptusöl, Kamillenöl, Zimtöl, Karottenöl, Zedernöl, Zitronenöl, Kopaivaöl, Zypressenöl, Elemiöl, Eukalyptusöl, Wacholderöl, Geranienöl, Gewürznelkenöl, Helichrysumöl, Ysopöl, Lavendelöl, Limettenöl, Muskatblütenöl, Muskatöl, Myrtenheideöl, Bitterorangenöl, Orangenöl, Majoranöl, Palmarosaöl, Grapefruitöl, Patschuliöl, Rosmarinöl, Rosenöl, Sandelholzöl, Bohnenkrautöl, Salbeiöl, Thymianöl, Ylang-Ylang-Öl, Kanada-Balsam, Peru-Balsam oder Tolu-Balsam ausgewählt ist/sind.

8. Verfahren nach einem der Ansprüche 1 bis 5, **dadurch gekennzeichnet, dass** man Probleme geröteter Haut behandelt, indem man ein essentielles Öl oder mehrere essentielle Öle verwendet, das/die unter den essentiellen Ölen Bittermandelöl, Lavendelöl, Basilikumöl, Benzoeöl, Rosenholzöl, Cadeöl, Eukalyptusöl, Kamillenöl, Zimtöl, Karottenöl, Zedernöl, Zitronenöl, Kapaivaöl, Zypressenöl, Elemiöl, Eukalyptusöl, Wacholderöl, Geranienöl, Gewürznelkenöl, Helichrysum-Öl, Ysopöl, Lavendelöl, Limettenöl, Muskatblütenöl, Muskatöl, Myrtenheideöl, Bitterorangenöl, Orangenöl, Majoranöl, Palmarosaöl, Grapefruitöl, Patschuliöl, Rosmarinöl, Rosenöl, Sandelholzöl, Bohnenkrautöl, Salbeiöl, Thymianöl und Ylang-Ylang-Öl und Kanada-Balsam, Peru-Balsum und Tolu-Balsam ausgewählt ist / sind.

9. Verfahren nach einem der Ansprüche 1 bis 5, **dadurch gekennzeichnet, dass** man Busenprobleme behandelt, indem man wie zur Straffung des Busens mehrere essentielle Öle verwendet, das/die unter den essentiellen Ölen Bittermandelöl, Lavendelöl, Basilikumöl, Benzoeöl, Rosenholzöl, Cadeöl, Eukalyptusöl, Kamillenöl, Zimtöl, Karottenöl, Zedernöl, Zitronenöl, Kopaivaöl, Zypressenöl, Elemiöl, Eukalyptusöl, Wacholderöl, Geranienöl, Gewürznelkenöl, Helichrysum-Öl, Ysopöl, Lavendelöl, Limettenöl, Muskatblütenöl, Muskatöl, Myrtenheideöl, Bitterorangenöl, Orangenöl, Oreganoöl, Palmarosaöl, Grapefruitöl, Patschuliöl, Rosmarinöl, Rosenöl, Sandelholzöl, Bohnenkrautöl, Salbeiöl, Thymianöl und Ylang-Ylang-Öl und Kanada-Balsam, Peru-Balsam und Tolu-Balsam ausgewählt ist/sind.

10. Kit zur kosmetischen Behandlung der Haut, der behaarten Kopfhaut und ihrer verwandten Teile zur Durchführung eines Verfahrens nach den Ansprüchen 1 bis 9, **dadurch gekennzeichnet, dass** es ein oder mehrere Gemische essentieller Öle und einen Generator (1) für athermische gepulste hochfrequente elektromagnetische Wellen umfasst, der fähig ist, eine Frequenz zwischen 1 MHz und 300 MHz mit einem Abstand von 0,1 bis 400 ms zwischen jeder Wellenpulsation mit einer Leistung von 10⁻⁶ W bis 2 W zu emittieren.

11. Kit zur kosmetischen Behandlung der Haut, der behaarten Kopfhaut und ihrer verwandten Teile nach Anspruch 10, **dadurch gekennzeichnet, dass** er ein oder mehrere Gemische essentieller Öle, einen Generator (1) für athermisch gepulste hochfrequente elektromagnetische Wellen, der fähig ist, eine Frequenz zwischen 1 MHz und 300 MHz mit einem Abstand von 0,1 bis 400 ms zwischen jeder Pulsation der Wellen mit einer Leistung von 10⁻⁶ W bis 2 W zu emittieren, und Instruktionen für die kombinierte Verwendung von essentiellen Ölen im Hinblick auf eine Verstärkung der Wirkungen umfasst.

## Claims

1. A non therapeutic method for amplification with synergetic effect of the standard effects of an essential oil with beneficent properties for the skin, the scalp, the nails and the hair, of pulsed high frequency electromagnetic waves, **characterized in that** the desired essential oil or mixture of essential oils are applied to the zone to be treated, and said zone is subjected to the action of athermal pulsed high frequency electromagnetic waves, with a frequency ranging between 1 MHz and 300 MHz, with a time spacing of 0.1 to 400 milliseconds between each wave impulse, and with a power of 10⁻⁶ to 400 milliseconds between each wave impulse, and with a power of 10⁻⁶ W to 2W.

2. A method according to claim 1, **characterized in that** the emission of the wave generator ranges between 0.2 and 1 mW.

3. A method according to one of claims 1 and 2, **characterized in that** the emission of the waves is achieved by means of one or more antennae fitted onto a device serving as a support for the antenna or antennae, said antennae being separated from the zone to be treated by between 0.5 and 15 cm.

4. A method according to one of claims 1 to 3, **characterized in that** the area of the antenna or antennae ranges between 50 and 300 cm².

5. A method according to one of claims 1 to 4, **characterized in that** the emission of the waves is effected less than 3 hours after the application of the essential oils.

6. A method according to one of claims 1 to 5, **characterized in that** the problem of hair loss are treated using one or more essential oils chosen form the essential oils of bay St Thomas, birch, cade, camomile, cinnamon, cedar, lemon, cypress, geranium, laurel, lavender, orange, patchouli, pine, rosemary, sandalwood, sage, wild thyme, thuya, thyme, or ylang-ylang.

7. A method according to one of claims 1 to 5, **characterized in that** problems of wrinkles are treated using one or more essential oils chosen from the essential oils of bitter almond, spike lavender, basil, benzoin, rosewood, cade, cajeput, camomile, cinnamon, carrot, cedar, copaiba, lemon, cypress, elemi, eucalyptus, juniper, geranium, clove, helichrysum, hyssop, lavender, lime, mace, nutmeg, niaouli, bitter orange, orange, oregano, palmarosa, grapefruit, patchouli, rosemary, rose, sandalwood, savory, sage, thyme, ylang-ylang, Canada balsam, balsam of Peru and balsam of Tolu.

8. A method according to one of claims 1 to 5, **characterized in that** problems of reddened skin are treated, using one or more essential oils chosen from the essential oils of bitter almond, spike lavender, basil, benzoin, rosewood, cade, cajeput, camomile, cinnamon, carrot, cedar, lemon, copaiba, cypress, elemi, eucalyptus, juniper, geranium, clove, helichrysum, hyssop, lavender, lime, mace, nutmeg, niaouli, bitter orange, orange, oregano, palmarosa, grapefruit, patchouli, rosemary, rose, sandalwood, savory, sage, thyme, ylang-ylang, Canada balsam, balsam of Peru and balsam of Tolu.

9. A method according to one of claims 1 to 5, **characterized in that** problems of the breasts are treated, as for firming of the bust, using one or more essential oils chosen from the essential oils of bitter almond, spike lavender, basil, benzoin, rosewood, cade, cajeput, camomile, cinnamon, carrot, cedar, lemon, copaiba, cypress, elemi, eucalyptus, juniper, geranium, clove, helichrysum, hyssop, lavender, lime, mace, nutmeg, niaouli, bitter orange, orange, oregano, palmarosa, grapefruit, patchouli, rosemary, rose, sandalwood, savory, sage, thyme, ylang-ylang, Canada balsam, balsam of Peru and balsam of Tolu.

10. A kit for cosmetic treatment of the skin, scalp and their derivatives for implementation of the method according to one of claims 1 to 9, **characterized in that** it includes one or more mixtures of essential oils, and a pulsed athermal high frequency electromagnetic wave generator (1) capable of emitting a frequency ranging between 1 MHz and 300 MHz, with a time spacing of 0.1 to 400 milliseconds between each wave impulse, with a power of 10⁻⁶ W to 2W.

11. A kit for cosmetic treatment according to claim 1, **characterized in that** it includes one or more mixtures of essential oils, a pulsed athermal high frequency electromagnetic wave generator (1) capable of emitting a frequency ranging between 1 MHz and 300 MHz, with a time spacing of 0.1 to 400 milliseconds between each wave impulse, with a power of 10⁻⁶ W to 2W, and instructions for joint use with essential oils to obtain amplified effects thereof.
